Europäisches Patentamt

European Patent Office

Office Européen des brevets

(19)

(11) Veröffentlichungsnummer : **0 240 581 B1**

# (12) EUROPÄISCHE PATENTSCHRIFT

(45) Veröffentlichungstag der Patentschrift :
02.01.91 Patentblatt 91/01

(51) Int. Cl.⁵ : **A61K 9/52**

(21) Anmeldenummer : 86104655.5

(22) Anmeldetag : 05.04.86

(54) Gelatinekapseln mit gesteuerter Wirkstofffreisetzung und Verfahren zur Herstellung derselben.

Die Akte enthält technische Angaben, die nach dem Eingang der Anmeldung eingereicht wurden und die nicht in dieser Patentschrift enthalten sind.

(43) Veröffentlichungstag der Anmeldung :
14.10.87 Patentblatt 87/42

(45) Bekanntmachung des Hinweises auf die Patenterteilung :
02.01.91 Patentblatt 91/01

(84) Benannte Vertragsstaaten :
AT BE CH DE FR GB IT LI LU NL SE

(56) Entgegenhaltungen :
DE-A- 2 627 113
DE-B- 1 295 989
US-A- 4 055 554
Tegge - Stärken Seite 171

(73) Patentinhaber : R.P. Scherer GmbH
Gammelsbacher Strasse 2 Postfach 1243
D-6930 Eberbach/Baden (DE)

(72) Erfinder : Fischer, Gerhard, Dr.
Hohenstaufenstrasse 28/1
D-6930 Eberbach/Baden (DE)

(74) Vertreter : Werner, Hans-Karsten, Dr. et al
Deichmannhaus am Hauptbahnhof
D-5000 Köln 1 (DE)

**Beschreibung**

Gegenstand der vorliegenden Erfindung sind Gelatinekapseln mit gesteuerter Wirkstofffreisetzung und Verfahren zur Herstellung derselben, wobei die Steuerung sowohl den Zeitraum als auch den Ort der Freisetzung der Wirkstoffe betrifft.

Bei einer Reihe von Arzneimitteln ist es erwünscht, daß sie den Wirkstoff erst nach einem bestimmten Zeitraum (Retardformen) oder aber erst im Dünndarm (magensaftresitent) freisetzen. So sind z.B. bei der Herstellung von Gelatinekapseln eine Reihe von Verfahren und Maßnahmen bekannt, sie zu retardieren oder magensaftresistent zu machen. Die älteste dieser Maßnahmen ist die nachträgliche Behandlung der fertigen Kapseln mit Formaldehyd. Ein Nachteil dieses Verfahrens besteht darin, daß die Härtung der Gelatine nur schwer steuerbar ist, da die Reaktivität des Formaldehyds auf die Gelatinehülle sehr unterschiedlich sein kann und vor allem stark zeitabhängig ist. In der Zwischenzeit ist Formaldehyd in den Verdacht geraten, kanzerogen zu sein, so daß ein völliges Verbot für die freie Benutzung dieses Stoffes zu befürchten ist.

Die DE-OS-26 27 113 beschreibt Kapseln zur kontrollierten Freigabe von Arzneimitteln, bei denen die Kapselhülle vernetzte Gelatine enthält, welche in basischen und sauren Körperflüssigkeiten unlöslich ist. Die Vernetzung wird an fertigen Gelatinekapseln durchgeführt. Die vernetzte Gelatine löst sich nicht mehr, sondern wirkt nur noch als Diffusionsmembran.

Die US-A-4,055,554 beschreibt die Härtung und Streckung von Gelatine mit Hilfe von Aldehydstärken, insbesondere Dialdehydpolysacchariden. Es wird weiterhin die Verbesserung der mechanischen Eigenschaften von Gelatine, die gewisse Mengen dieser Aldehydstärken enthält, beschrieben.

Die DE-B-12 95 989 beschreibt ein Verfahren zur Herstellung von Kollagenhüllen für Wurstmassen, bei dem die Naßreißfestigkeit und die Festigkeit während des Räucher- und Spülungsvorganges erhöht wird.

Aus der DE-OS 30 15 530 ist ein Verfahren zur Herstellung magensaftresistenter Gelatinekapseln bekannt, bei dem die Kapselmasse oder die vorgebildeten Kapseln mit einer Lösung von Phthalsäurealdehyden behandelt werden. Angeblich soll sich hierbei die aldehydische Gruppe der Phthalsäurealdehyde mit der Gelatine verbinden und eine feste Verbindung mit dem Gelatineeiweiß herstellen. Der Säureanteil der Verbindungen soll ein Schutz gegenüber einer Auflösung im sauren Medium des Magens bewirken und andererseits eine rasche und vor allem auch steuerbare Auflösung der Gelatine im neutralen Medium des Darms bewirken. Vorzugsweise wurden vorgebildete Weichgelatinekapseln oder Hartgelatinekapseln in noch ungetrocknetem Zustand mit einer ethanolischen Lösung des Phthalsäurealdehyds behandelt. Angeblich wurde auch Phthalsäureanhydrid in Lösung mit der Gelatinemasse gemischt und die Masse zu Gelatinekapseln verarbeitet. Dieses Verfahren ist in der Praxis nie zur Anwendung gekommen, da Phthalsäurealdehyde hoch toxisch sind. Da die Reaktion zwischen Phthalsäurealdehyden und Gelatine nie quantitativ erläuft und die Bindung an die Gelatine auch reversibel sein kann, muß damit gerechnet werden, daß die toxischen Phthalsäurealdehyde in freier Form in den Körper gelangen.

Eine weitere Möglichkeit, retardierte oder magensaftresistente Kapseln zu erhalten, besteht in den sogenannten Lackier- oder Coating-Verfahren. Hierzu werden beispielsweise Polymere, die sich unterhalb des pH-Wertes von 5 oder 5,5 nicht lösen, in organischen Lösungsmitteln gelöst und schichtweise so lange aufgetragen, bis eine genügende Schichtdicke erreicht ist, die dem Magensaft mindestens 2 Stunden widersteht. Für diesen Zweck geeignete Polymere sind z.B. Celluloseacetatphthalat und Hydroxypropylmethylcellulosephthalat. Als Lösungsmittel werden Alkohole oder chlorierte Kohlenwasserstoffe oder Mischungen derselben ggf. unter Zusatz von Weichmachern verwendet. Dieses Verfahren ist apparativ und zeitlich aufwendig. Durch die verdampfenden organischen Lösungsmitteln entstehen weitere Probleme.

In gleicher Weise lassen sich auch wasserunlösliche Polymere wie z.B. Ethylcellulose aufbringen. Ein derartiger Polymerfilm stellt theoretisch eine Diffusionsbarriere für den in der Gelatinekapsel eingebrachten Wirkstoff dar. In der Praxis kann dieses Verfahren jedoch nicht angewandt werden, da bisher noch kein physiologisch unbedenkliches Polymeres gefunden wurde, welches dem Quellungsdruck der Gelatinehülle standhält und daher nicht bereits nach kurzer Zeit reißt.

Die Erfindung hat sich daher die Aufgabe gestellt, die Steuerung der Wirkstofffreigabe in anderer, einfacher und unbedenklicher Form durchzuführen und dabei reproduzierbar zu guten und gleichmäßigen Ergebnissen zu kommen.

Es wurde gefunden, daß diese Aufgabe überraschend einfach gelöst werden kann durch einen Gehalt von 0,001 bis 10 Gew.-%, vorzugsweise 0,01 bis 2 Gew.-% physiologisch und toxikologisch unbedenklicher Aldehyde mit mindestens 4 Kohlenstoffatomen, nämlich Terpene, Zimtaldehyd, Aldosen oder Gemische derselben. Besonders bewährt haben sich etherische Öle mit einem hohen Gehalt an Aldehyden wie Citral oder Citral in reiner Form, Zimtaldehyd sowie die Aldosen, Xylose und Arabinose.

Erstaunlicherweise ist es möglich, diese Aldehyde dem Gelatinesud zuzugeben und diesen dann in an sich bekannter Weise zu Kapseln zu verarbeiten, ohne daß es dabei zu Störungen kommt. Von Formaldehyd und Glutardialdehyd ist nämlich bekannt, daß sie bereits bei Zugabe zum Gelatinesud mit der Gelatine so stark reagieren, daß es zu einem starken Anstieg der Viskosität kommt, der die technische Weiterverarbeitung stört oder sogar unmöglich macht. Überraschenderweise sind die erfindungsgemäß verwendeten Aldehyde mit mindestens 4 Kohlenstoffatomen in der Lage bereits in relativ geringen Mengen die Eigenschaften der fertigen Gelatinekapsel zu verändern, ohne daß es dabei zu Schwierigkeiten bei der Verarbeitung des Gelatinesudes kommt. Dies ist um so erstaunlicher, als der Gelatinesud auf Temperaturen von mindestens 50°C gebracht und gehalten werden muß, die Trocknung hingegen bei niedrigeren Temperaturen bis hin zur Raumtemperatur erfolgt.

Das erfindungsgemäße Verfahren zur Herstellung von Gelatinekapseln mit gesteuerter Wirkstofffreisetzung ist somit dadurch gekennzeichnet, daß dem Gelatinesud 0,001 bis 10 Gew.-%, vorzugsweise 0,01 bis 2 Gew.-%, physiologisch und toxikologisch unbedenklicher Aldehyde mit mindestens 4 Kohlenstoffatomen, nämlich Terpene, Zimtaldehyd, Aldosen oder Gemische derselben zugegeben werden und der Sud in an sich bekannter Weise zu Kapseln verarbeitet wird.

Durch den Zusatz der erfindungsgemäß verwendeten Aldehyde ist es möglich, je nach Art und Menge eine leichte Retardierung, eine starke Retardierung oder eine Magensaftbeständigkeit zu erreichen.

Die Steuerung der Wirkstofffreisetzung ist erfindungsgemäß sowohl bei Weichgelatinekapseln als auch bei Hartgelatinekapseln möglich.

Ein besonderer Vorteil erfindungsgemäß hergestellter Gelatinekapseln ist die gute Reproduzierbarkeit der erzielten Ergebnisse, wobei im Gegensatz zur nachträglichen Behandlung der Gelatine mit Formaldehyd der Zeitfaktor offensichtlich keine Rolle mehr spielt. Dies ist wahrscheinlich darauf zurückzuführen, daß die Aldehyde bereits unmittelbar nach der Zugabe zum Gelatinesud bei den erhöhten Temperaturen mit der Gelatine reagieren, die Vernetzung jedoch erst in der zweiten Phase des Trocknens stattfindet. Da es sich obendrein um physiologisch und toxikologisch unbedenkliche Aldehyde handelt, würden auch nicht umgesetzte Reste oder wieder abgespaltene Reste der Aldehyde unschädlich sein. Da keine organischen Lösungsmittel zur Anwendung kommen, ist das erfindungsgemäße Verfahren bezüglich Arbeitssicherheit und Umweltbelastung besonders vorteilhaft.

In den folgenden Beispielen ist die Erfindung näher erläutert.

## Beispiel 1

Zu einer Weichkapselhüllenformulierung bestehend aus 46 Teilen Gelatine, 20 Teilen Glycerin und 34 Teilen Wasser, werden 0,5 Teile Zimtaldehyd in 2,5 Teile 1,2 Propandiol gegeben. Diese Formulierung wird in an sich bekannter Weise zu Weichgelatinekapseln weiterverarbeitet. Nach dem Auftrocknen werden Kapseln erhalten, die nach ca. 30 bis 45 Minuten den Kapselinhalt freigeben.

## Beispiel 2

Gemäß Beispiel 1 werden zur gleichen Weichkapselhüllenformulierung 1 Teil Citral und 5 Teile 1,2 Propandiol gegeben. Nach dem Auftrocknen werden Kapseln erhalten, die sich in künstlichem Magensaft innerhalb 2 Stunden nicht auflösen, jedoch in künstlichem Darmsaft innerhalb 1 Stunde löslich sind.

## Beispiel 3

Weichkapselhüllenformulierung gemäß Beispiel 1 mit 2 Teilen Xylose zu 100 Teilen Gelatinemasse. Die trockene Kapselhülle ist in künstlichen Körpersäften nur noch quellfähig und stellt daher eine Diffusionsmembran dar, aus der die Wirkstoffe langsam hindurchdiffundieren können.

## Beispiel 4

Zu einer Hartkapselformulierung bestehend aus 33 Teilen Gelatine und 66 Teilen Wasser werden 2 Teile Arabinose gegeben. Nach Herstellung der Hartgelatinekapseln werden diese mit Pulver befüllt. Die pulverbefüllten Hartkapseln werden 2 Stunden in künstlichem Magensaft exponiert. Die Kapselhülle quillt lediglich auf, ohne das Pulver, das umgebende Medium, freizugeben. Nach Wechsel des Prüfmediums in künstlichen Darmsaft, löst sich die Hülle innerhalb einer halben Stunde und gibt den Kapselinhalt frei.

## Ansprüche

1. Gelatinekapseln mit gesteuerter Wirkstofffreisetzung gekennzeichnet durch einen Gehalt von 0,001 bis 10 Gew.-%, vorzugsweise 0,01 bis 2 Gew.-% physiologisch und toxikologisch unbedenklicher Aldehyde mit mindestens 4 Kohlenstoffatomen, nämlich Terpene, Zimtaldehyd, Aldosen oder Gemische derselben.

2. Verfahren zur Herstellung von Gelatinekapseln mit gesteuerter Wirkstofffreisetzung, dadurch gekennzeichnet, daß dem Gelatinesud 0,001 bis 10 Gew.-%, vorzugsweise 0,01 bis 2 Gew.-% physiologisch und toxikologisch unbedenklicher Aldehyde mit mindestens 4 Kohlenstoffatomen, nämlich Terpene, Zimtaldehyd, Aldosen oder Gemische derselben zugegeben werden und der Sud in an sich bekannter Weise zu Kapseln verarbeitet wird.

## Claims

1. Gelatin capsules for the controlled release of the active ingredient, characterized by a content of from 0.001 to 10% by weight, and preferably of from 0.01 to 2% by weight, of physiologically and toxicologically acceptable aldehydes having at least 4 carbon atoms, namely terpenes, cinnamaldehyde, aldoses or mixtures thereof.

2. A process for the preparation of gelatin capsules for the controlled release of the active ingredient, characterized in that the gelatin decoction is admixed with from 0.001 to 10% by weight, and preferably with from 0.01 to 2% by weight, of physiologically and toxicologically acceptable aldehydes having at least 4 carbon atoms, namely terpenes, cinnamaldehyde, aldoses or mixtures thereof, and the resulting decoction is processed in a per se known manner to form capsules.


## Revendications

1. Capsules de gélatine à libération contrôlée de matière active, caractérisées par une teneur de 0, 001 à 10% en poids, de préférence 0, 01 à 2% en poids d'aldéhydes physiologiquement et toxicologiquement neutres, ayant au moins 4 atomes de carbone, en particulier des terpènes, de l'aldéhyde cinnamique, des aldoses, ou des mélanges de ces corps.

2. procédé d'obtention de capsules de gélatine à libération contrôlée de matière active, caractérisé en ce qu'on ajoute à la décoction de gélatine 0,001 à 10% en poids, de préférence 0,01 à 2% en poids d'aldéhydes physiologiquement et toxicologiquement neutres, ayant au moins 4 atomes de carbone, en particulier des terpènes, de l'aldéhyde cinnamique, des aldoses, ou des mélanges de ces corps, et on transforme, de façon connue en soi, la décoction en capsules.